# EUROPÄISCHE PATENTSCHRIFT

(11) **EP 2 029 212 B1**
(45) Veröffentlichungstag und Bekanntmachung des Hinweises auf die Patenterteilung: **30.05.2012**
(21) Anmeldenummer: 07720157.2
(22) Anmeldetag: 22.05.2007
(51) Int. Cl.: A61M 25/00

(54) **KATHETERSPITZE**
CATHETER TIP
EMBOUT DE CATHÉTER

(30) Priorität: 07.06.2006 CH 921062006
(43) Veröffentlichungstag der Anmeldung: 04.03.2009
(73) Patentinhaber: Schwager Medica, 8404 Winterthur (CH)
(72) Erfinder: SCHWAGER, Michael, 8404 Winterthur (CH)
(74) Vertreter: Roshardt, Werner Alfred
(86) Internationale Anmeldenummer: PCT/CH2007/000260
(87) Internationale Veröffentlichungsnummer: WO 2007/140635

(56) Entgegenhaltungen:
- EP-A1- 0 530 970
- EP-A2- 0 808 637
- WO-A-03/004085
- US-A- 4 636 346
- US-A- 5 957 893
- US-A1- 2003 120 207
- US-A1- 2003 139 760
- US-A1- 2005 131 445
- US-A1- 2005 171 591
- US-B1- 6 171 295

## Beschreibung

### Technisches Gebiet

Die Erfindung betrifft eine Katheterspitze mit einer Vorderseite und einer Rückseite, welche auf einen Katheter aufsetzbar ist, wobei die Katheterspitze zwei im Wesentlichen zylindrisch ineinander liegende Schichten mit einer dünnen Bindezwischenschicht (14) umfasst.

### Stand der Technik

Katheter sind Röhrchen oder Schläuche verschiedenen Durchmessers, zum Beispiel aus Kunststoff, Latex, Silikon, Metall oder Glas. Mit ihnen werden röhrenförmige Körperflüssigkeiten führende Hohlorgane, nachfolgend kurz Organ(e) genannt, zum Beispiel Harnröhre, Speiseröhre, Gallengänge, Gefässe und blutführende Adern wie Herzarterien, von Mensch und/oder Tier, sondiert, durchstossen, entleert, gefüllt und/oder gespült. Dies geschieht aus diagnostischen (untersuchungsbedingten) oder therapeutischen (behandlungsbedingten) Gründen.

In den letzten Jahren entwickelten sich die Kathetermaterialien rasch. Wurden früher nur relativ unflexible Materialien wie Metall oder Glas verwendet, kam später Gummi hinzu, der heute zunehmend durch andere flexible Stoffe, wie Latex, Polyvinylchlorid (PVC) oder Silikon, ersetzt wird. Katheter der neusten Generation werden zudem häufig beschichtet, um das Einführen durch bessere Gleiteigenschaften für den Patienten angenehmer zu machen und/oder um bei Kathetern mit Langzeitanwendung die Bildung eines Bakterienbiofilms zu unterdrücken.

Ohne auf die Vielzahl der bekannten Katheterarten beziehungsweise -typen im Einzelnen einzugehen, können wichtige Unterscheidungskriterien für Katheter beispielsweise folgendermassen zusammengefasst werden:
- Anwendungsart: Mensch oder Tier, diagnostisch oder therapeutisch
- betroffenes Organ: Harnröhre, Speiseröhre, Gallengänge, Gefässe, Adern, Herzarterien
- Verwendetes Material: Metalle, Legierungen, Glas, Gummi, Kunststoffe, Latex, PVC, Silikon
- Materialeigenschaften: Flexibilität, Festigkeit, Gleitfähigkeit
- Nutzungshäufigkeit: Einmalige oder mehrmalige Nutzung
- Einsatzdauer: Kurzzeit- oder Langzeitanwendung
- Fixierung: Selbst fixierend (zum Beispiel mittels eines Ballons) oder nicht
- Geschlechterspezifische Bauform: Für weibliche, männliche oder für beide Geschlechter geeignet
- Bauform im Allgemeinen: Einkanal-/Mehrkanalkatheter, Anzahl und Anordnung der Öffnungen; gerade/gebogen, mit/ohne Erweiterung, unbeschichtet/beschichtet, mit/ohne Wandverstärkung, Einführung und Anwendung ohne/mit Hilfsmittel (zum Beispiel Spreiz- und/oder Gleitmittel)

Eine zentrale Anforderung an einen Katheter ist ein möglichst kleiner Aussendurchmesser mit möglichst kleinen Wandstärken. Gleichzeitig müssen die Gleit- und Flexibilitätseigenschaften möglichst gut und die Reiss- und/oder Stauchgefahr während des Einführvorgangs klein sein. Die Kombination aus geringem/r Aussendurchmesser/Wandstärke bei gleichzeitig sehr guten Gleit- und Flexibilitätseigenschaften ermöglicht einen vergleichsweise geringen Widerstand beim Einführen des Katheters in das betreffende Organ. Dies auch deswegen, weil sich ein flexibler Katheter dem Verlauf eines Organs, in das er eingeführt wird, anpasst.

Diese Anforderungen gelten wenigstens in gleichem Masse auch für Katheterspitzen, welche nach dem Stand der Technik durch einen kleineren Aussendurchmesser im Bereich der Spitze einem Führungsdraht folgend das Einführen eines Katheters vorspurt und damit erleichtert. Katheterspitzen können auch auf einen Katheter aufsetzbar ausgebildet sein. Aufsetzbare Katheterspitzen erlauben eine von den restlichen Katheterteilen/-elementen unabhängige Herstellung, wodurch die Anforderungen bezüglich der Gleit-/ Flexibilitätseigenschaften und/oder der Reiss-/Staucheigenschaften weiter optimiert werden können. Auch geringfügige Verbesserungen der genannten Eigenschaften sind von grossem Nutzen und entsprechender Bedeutung.

Die US 4,636,346 offenbart einen Führungskatheter mit einem gleitfähigen Lumen, welches im Bereich des Katheterkörpers eine dreischichtige und im Bereich der Katheterspitze eine zweischichtige Struktur aufweist. Bei den drei Schichten im Bereich des Katheterkörpers handelt es sich um eine innere, gleitfähige Schicht, auf welche eine dünne Zwischenschicht hoher Festigkeit aufgebracht ist, sowie um eine flexible dünnwandige Aussenschicht. Der Katheterkopf 23 kann separat und am Katheterkörper (z. B. durch Verschweissen) befestigbar ausgebildet sein.

Die US 2005/171591 A1 beschreibt Katheter, insbesondere zum Einbringen und Positionieren von Stents. Am Katheter ist eine Katheterspitze durch eine Klebung, eine thermische Verbindung oder eine Schweissung befestigt. Sie kann Ausbuchtungen oder zurückgenommene Abschnitte umfassen, ihre Länge kann zwischen 4 und 70 mm betragen. Die Katheter können zudem im Bereich der Spitze radioopake Marker, z. B. in Form eines umlaufenden Rings und z. B. aus Platin, aufweisen.

Die US 2005/131445 A1 betrifft Katheterspitzen und die Verbindung von zueinander inkompatiblen Elementen von Kathetern. Unter anderem wird eine aufsetzbare Katheterspitze offenbart, welche mit dem Katheter verschweisst werden kann. Die Länge der Katheterspitze (Überhang über den Katheter) beträgt zwischen 0 und 7 mm. Die Spitze kann abgeschrägt sein. Die Katheterspitze oder ein Teil davon kann als Verbindungsschicht zum Verbinden des Ballonkörpers mit dem inneren Schaft eingesetzt werden und wird zu diesem Zweck mit Vorteil aus Plexar hergestellt. Die innere Schicht des Katheterschafts kann mit einer gewindeartigen Struktur versehen sein.

Die US 2003/120207 A1 befasst sich mit der Verbindung von Polymerschichten im Rahmen der Herstellung von Kathetern, insbesondere der Verbindung eines Katheterballons und einem distalen Abschnitt des Katheters. Dabei wird vorgeschlagen, dass eine erste Schicht aus beispielsweise hochdichtem Polyethylen mit einer zweiten Schicht aus beispielsweise Nylon mittels einer Zwischenschicht aus Plexar verbunden werden könne.

Die WO 03/004085 A2 betrifft intravaskuläre Katheter mit mehrschichtigen Katheterspitzen. Die Spitzen sind insbesondere dreischichtig, wobei die mittlere Schicht aus einem Polymermaterial gebildet ist, das einen hohen Prozentanteil eines radioopaken Zusatzes enthält und wobei die innere und die äussere Schicht aus einfach verbindbaren Materialien bestehen, insbesondere Polyether-Block-Amiden. Die Schichtdicken der drei Schichten betragen je zwischen 0.025 und 0.13 mm. Die Katheterspitze ist thermisch mit dem Katheterkörper verbunden.

Die EP 0 808 637 A2 offenbart einen Katheter mit einer mehrschichtig ausgebildeten Katheterspitze. Die Spitze umfasst eine äussere Schicht und eine innere Versteifungsschicht, zwischen denen ein metallisches Geflecht angeordnet ist. Zwischen der metallischen Schicht und der äusseren Schicht kann ein Klebstoff vorhanden sein. In die innere Versteifungsschicht können radioopake Marker, z. B. ringförmige Marker aus Platin, eingebettet sein.

Die US 6,171,295 B1 zeigt einen verstärkten Katheter. Im Bereich der Katheterspitze kann ein radioopaker Marker vorhanden sein, welcher eine Verstärkungsschicht umschliesst und seinerseits von einer atraumatischen Spitzenschicht umgeben ist. Die Verstärkungsschicht umschliesst wiederum eine innere, gleitfähige Schicht, z. B. aus Teflon, wobei zwischen der Verstärkungsschicht und der inneren Schicht eine Verbindungsschicht mit einer Dicke von ca. 7.6 µm angeordnet sein kann

Die US 2003/139760 A1 betrifft Dilatationskatheter, welche eine atraumatische Katheterspitze umfassen können. Die Spitze hat eine Länge von 2-6 mm und kann sowohl stirnitig verbunden, in den Katheter eingeführt oder auf diesen aufgeschoben werden. Die Verbindung zwischen Spitze und Katheter erfolgt durch eine Verschweissung. Aufgrund der vielfältigen Anforderungen aus Medizin und/oder Forschung ist es wünschenswert, Katheterspitzen bezüglich der genannten Eigenschaften weiter zu verbessern.

### Darstellung der Erfindung

Aufgabe der Erfindung ist es, eine dem eingangs genannten technischen Gebiet zugehörende Katheterspitze zu schaffen, welche im Vergleich zu bisherigen Katheterspitzen
- bei gleicher Flexibilität einer erhöhten Druckfestigkeit widersteht,
- bei gleicher Druckfestigkeit eine erhöhte Flexibilität hat, oder
- gleichzeitig eine erhöhte Flexibilität und Druckfestigkeit hat.

Die Lösung der Aufgabe ist durch die Merkmale des Anspruchs 1 definiert. Weitere Vorteile werden durch die Merkmale der abhängigen Ansprüche erreicht. Gemäss der Erfindung umfasst die Katheterspitze zwei im Wesentlichen zylindrisch ineinander liegende Schichten mit einer dünnen Bindezwischenschicht. Spezielle und weiterbildende Ausführungsformen sind Gegenstand von abhängigen Patentansprüchen.

Überraschend hat sich gezeigt, dass sich die Gleit-/Flexibilitätseigenschaften und/oder die Reiss-/Staucheigenschaften einer Katheterspitze gegenüber dem Stand der Technik weiter verbessern, wenn die Katheterspitze dreischichtig ausgeführt ist.

### Vorteilhaft besteht dabei

- die innere Schicht aus einem Werkstoff hoher Druckfestigkeit, vorzugsweise aus High Density Polyethylen (HDPE) oder Teflon,
- die Bindezwischenschicht aus einem Werkstoff mit guten Adhäsionseigenschaften, vorzugsweise aus Plexar, und/oder
- die äussere Schicht aus einem flexiblen, aber nur gering dehnbaren Werkstoff, vorzugsweise aus Polyamid, Nylon und/oder Polyester.

Im Vergleich zum Stand der Technik können die wesentlichen Vorteile der erfindungsgemässen Katheterspitze folgendermassen zusammengefasst werden:
- Die Katheterspitze erhöht bei gleichen geometrischen Aussenmassen gleichzeitig die Flexibilität und die Druckfestigkeit, wobei Gleitfähigkeit und Reissfestigkeit der Katheterspitze im Vergleich zum Stand der Technik wenigstens erhalten bleiben.
- Weil sich gegenüber dem Stand der Technik die gleiche Flexibilität und Druckfestigkeit bei kleineren geometrischen Aussenabmessungen realisieren lassen, wird Platz für weitere Verwendungen geschaffen. Konkret ist in einer Ausführungsvariante ein weiteres, freies Lumen in die Katheterspitze eingesetzt, wodurch sich bei gegenüber dem Stand der Technik ähnlichen geometrischen Aussenabmessungen zusätzlich die Möglichkeit ergibt, ein flüssiges und/oder gasförmiges Medium, insbesondere eine Lösung und/oder eine Stammzelle vor der Katheterspitze in das betreffende Organ einzuführen beziehungsweise zu applizieren.
- Weil die Katheterspitze aufsetzbar geformt ist, kann sie in einem von den restlichen Katheterteilen/-elementen unabhängigen Verfahren hergestellt werden.
- Wird auf ein zusätzliches, freies Lumen verzichtet, lassen sich die geometrischen Aussenabmessungen im Vergleich zum Stand der Technik verkleinern.

Da die unterschiedlichen Materialien schlecht miteinander verbunden werden können, hält die äusserste Schicht der erfindungsgemässen Katheterspitze die inneren Schichten zusammen. Die Dicke der äusseren Schicht beträgt etwa 80 % und jene der inneren Schicht etwa 20 % der Gesamtschichtdicke.

Die dazwischen liegende Bindezwischenschicht ist dagegen sehr dünn und prozentual zur Gesamtdicke vernachlässigbar klein. Die Bindezwischenschicht der erfindungsgemässen Katheterspitze weist vorteilhaft eine Dicke von 1 bis 10 µm, vorzugsweise etwa 2 µm, auf.

Die Katheterspitze weist in einer vorteilhaften Ausführung vorzugsweise eine Länge von 0,5 bis 10 mm, insbesondere 3 bis 5 mm, auf.

Die Katheterspitze ist vorteilhaft nach vorne schräg zulaufend ausgebildet, wobei ein Aussendurchmesser an der Vorderseite vorzugsweise 0,35 bis 0,5 mm, insbesondere etwa 0,45 mm, und/oder ein Aussendurchmesser an der Rückseite vorzugsweise 0,45 bis 0,6 mm, insbesondere etwa 0,5 mm, beträgt.

Die Katheterspitze umfasst in einer vorteilhaften Variante wenigstens ein freies Lumen zum Einbringen eines flüssigen und/oder gasförmigen Mediums.

Der Durchmesser des freien Lumens beträgt im Bereich der Äustrittsöffnung vorzugsweise 0,05 bis 0,42 mm, insbesondere etwa 0,35 mm.

In weiteren Ausführungsformen ist die Katheterspitze mit einem über die Länge abschnittsweise konstanten oder veränderlichen, kreisförmigen, ovalen und/oder elliptischen Quer schnitt ausgebildet.

Weitere Ausführungsformen der Katheterspitze zeichnen sich dadurch aus, dass wenigstens eine Schicht und/oder ein Führungsdraht wenigstens teilweise gefärbt, reflektierend und/oder fluoreszierend ausgebildet ist. Damit ist es möglich, den Verlauf beziehungsweise die Position einer Katheterspitze im Körper eines Menschen und/oder eines Tieres sowohl während des Einführens als auch im eingeführten Zustand, zum Beispiel mit einem Röntgen- oder einem Ultraschallgerät, nachzuvollziehen.

Der Führungsdraht selbst ragt beim Einführen des Katheters über die Katheterspitze hinaus und folgt dank seines geringen Widerstands infolge des kleinen Durchmessers und dessen Flexibilität dem Verlauf des Organs. Dabei folgt die Katheterspitze dem Führungsdraht und schmiegt sich dem Verlauf des betreffenden Organs an. Der Führungsdraht ist aus gut gleitfähigem, flexiblem und gleichzeitig zähem Material gefertigt, vorzugsweise aus rostfreiem Stahl, Aluminium oder aus Kunststoffen mit hoher mechanischer Festigkeit, zum Beispiel PVC, Polyamid oder Polyester.

In einer vorteilhaften Variante ist nur die innerste Schicht gegenüber dem Führungsdraht gleitfähig ausgebildet, das heisst, die drei einzelnen Schichten sind zueinander vorteilhaft nicht gleitfähig ausgebildet. Die vorliegende Erfindung funktioniert grundsätzlich auch ohne den flexiblen Führungsdraht, zum Beispiel, wenn die Katheterspitze in ein Organ eingeführt wird, welches vergleichsweise kurz und/oder geradlinig ist.

In einer weiteren Ausführungsform ist die Katheterspitze weich und/oder im Bereich des Katheters vorzugsweise einschichtig ausgeführt, um eine stoff- und/oder kraftschlüssige Verbindung zwischen Katheterspitze und Katheter zu unterstützen.

In einem Verbindungsbereich zum Katheter, welcher vorzugsweise als Ballonkatheter und/oder als Monorail-Katheter ausgeführt ist, wird
- die Katheterspitze vorteilhaft stirnflächig zum Katheter verbindbar ausgeformt, und/oder
- die Katheterspitze in den Katheter und/oder der Katheter in die Katheterspitze einschiebbar ausgebildet.

Unter einem Ballonkatheter wird ein Kathetertyp in Kombination mit einem ein- oder mehrschichtig ausgeführten Ballon verstanden. Dieser Ballon kann mit einem flüssigen und/oder mit einem gasförmigen Medium gefüllt und entleert werden und dient dazu, den Katheter im betreffenden Organ eines Menschen/eines Tieres zu fixieren, dieses abzudichten und/oder aufzuweiten.

Unter einem Monorail-Katheter ist ein Kathetertyp zu verstehen, der mit einem Führungsdraht versehen durch eine einzige Person bedienbar beziehungsweise einführ- und herausziehbar ist.

Die erfindungsgemässe Katheterspitze ist im Verbindungsbereich zum Katheter vorteilhaft verschweissbar, verklebbar, pressbar und/oder pressschmelzbar ausgeführt.

In einer weiteren bevorzugten Variante weist die Katheterspitze in einem Bereich vor dem Verbindungsbereich mit dem Katheter eine gewölbte Ausbuchtung auf. Dabei haben sich insbesondere Ausbuchtungen, welche tonnenförmig ausgestaltet sind, als vorteilhaft erwiesen. Unter tonnenförmig wird hier verstanden, dass der Aussendurchmesser und/oder der Innendurchmesser der Katheterspitze in einem Bereich vor der Ausbuchtung zur Ausbuchtung hin kontinuierlich zunimmt und in einem Bereich hinter der Ausbuchtung kontinuierlich wieder abnimmt. Der Querschnitt der Katheterspitze ist dabei bevorzugt im wesentliche kreisförmig oder oval. Es hat sich gezeigt, dass durch die wölbende Ausformung eine verbesserte Knickstabilität des vorderen und meist sehr dünnen Bereichs der Katheterspitze erhalten wird. Gleichzeitig ist auch eine gute Einführbarkeit in Hohlorgane gewährleistet, da die wölbende Ausformung ein sanftes und wirkungsvolles Aufweiten von Verengungen in Hohlorganen ermöglicht. In Verbindung mit dem Schichtaufbau der erfindungsgemässen Katheterspitzen werden somit optimale mechanische Eigenschaften erhalten. Prinzipiell lassen sich aber auch nicht erfindungsgemässe Katheterspitzen mit einer derartigen gewölbten Ausbuchtung herstellen.

Es hat sich zudem als vorteilhaft erwiesen, die Ausbuchtung so auszugestalten, dass der Aussendurchmesser im Bereich der Ausbuchtung 5-25 %, insbesondere 10-20 %, grösser ist, als ein an der Vorderseite vorliegender Aussendurchmesser der Katheterspitze. Ein derartiger Wert stellt sicher, dass die Querschnittsfläche der Katheterspitze im Bereich der Ausbuchtung nicht zu gross ist, gleichzeitig aber eine optimale Knickstabilität erhalten wird.

In einer weiteren Variante weist die äussere Schicht der Katheterspitze im Bereich einer äusseren Oberfläche bzw. einer Mantelfläche eine um die Katheterspitze gewundene schraubenartige Struktur auf. Damit lässt sich der Katheter beispielsweise durch Drehbewegungen leichter vorwärts schieben, da die Drehbewegungen in vorwärts gerichteten Längskräften auf die Katheterspitze resultieren. Zudem wurde gefunden, dass die schraubenartige Struktur der äussersten Schicht sowohl die Knickstabilität als auch die Flexibilität der Katheterspitzen erhöht. Eine derartige Ausformung hat sich insbesondere bei den schichtartig aufgebauten und erfindungsgemässen Katheterspitzen als vorteilhaft erwiesen. Es ist prinzipiell aber auch möglich, Spitzen, welche beispielsweise lediglich aus einer einzelnen Schicht bestehen mit einer schraubenartigen Struktur zu versehen.

In einer bevorzugten Variante verläuft die schraubenartige Struktur von der Vorderseite beginnend in einer Richtung zur Rückseite der Katheterspitze hin und weist bevorzugt eine Länge von wenigstens 2 mm, insbesondere wenigstens 3 mm, auf. Damit steht die Wirkung der schraubenartigen Struktur im vordersten Bereich der Katheterspitze zur Verfügung. Eine derartige Katheterspitze ist insbesondere geeignet, wenn sehr starke Verengungen in den Hohlorganen überwunden werden müssen, da der vorderste Teil der Spitze den Katheter quasi durch die Verengung durchzieht.

Mit Vorteil weist die schraubenartige Struktur eine Gewindesteigung von 0,1-5 mm, insbesondere 0,8-1,5 mm auf.

Bei einer anderen vorteilhaften Variante ist die schraubenartige Struktur meist an dem etwas dickeren Verbindungsbereich von Katheterspitze und Katheter angebracht. Der vorderste, d. h. vor dem Verbindungsbereich liegende, Abschnitt der Katheterspitze ist in diesem Fall beispielsweise als ein im Wesentlichen zylinderischer Schaft ausgestaltet. Derartige Katheter eignen sich beispielsweise, wenn die Verengungen in einem Hohlorgan nicht besonders eng sind. Der vorderste Teil der katheterspitze kann in diesem Fall problemlos durch die Verengung durchgestossen werden. Sobald der etwas dickere Teil der Katheterspitze mit der schraubenartigen Struktur in die Verengung gelangt und darin anstösst, kann zusätzlich zur Stossbewegung eine Drehbewegung erzeugt werden, welche den problematischen dickeren Teil der Katheterspitze wie oben beschrieben durch die Verengung hindurchbewegt.

Von Vorteil sind an der Katheterspitze ein oder mehrere röntgendichte Marker angebracht. Damit lässt sich die Position der Katheterspitze in einem menschlichen oder tierischen Körper beispielsweise durch Röntgenabsorption jederzeit bestimmen.

Bevorzugt sind die Marker dabei in die äussere Schicht der Katheterspitze eingebettet. Damit ist sichergestellt, dass die Marker keine zusätzlichen Widerstandsflächen bieten, welche die Einführbarkeit der Katheterspitze vermindern würden. Dies kann beispielsweise dadurch erreicht werden, dass die äusserte Schicht der Katheterspitze von der Vorderseite her im Bereich vor dem Marker eine kontinuierlich zunehmende Dicke aufweist, welche direkt vor dem Marker ungefähr der Dicke Markers entspricht.

Besonders vorteilhaft ist der Abstand der Marker von der Vorderseite der Katheterspitze wenigstens gleich 5 Mal, bevorzugt 10 Mal, so gross, wie ein vorderer Innendurchmesser an der Vorderseite der Katheterspitze. Damit ist sichergestellt, dass der Marker nicht bereits im vordersten Bereich als Widerstandsfläche beim Einführen der Katheterspitze wirkt, was ein Einfädeln der Katheterspitze durch starke Verengungen erheblich erschweren würde. Aufgrund der weiter hinten angebrachten Marker steht der vorderste und optimal ausgeformte, dünne Teil der Katheterspitze hierfür vollumfänglich zur Verfügung. Bei Markern, welche wie vorstehend beschrieben in die äusserste Schicht eingebettet sind, wird zudem sichergestellt, dass die abfallende Flanke vor den Markern eine ausreichend flache Steigung aufweist, was wiederum die Einführbarkeit verbessert.

Von Vorteil enthalten die röntgendichten Marker Platin und weisen bevorzugt ein Volumen von 0,10-0,20 mm³ auf. Damit ist sichergestellt, dass ausreichend röntgendichtes Material vorliegt, welches durch Röntgenabsorption mit ausreichender Ortsauflösung detektiert werden kann.

In einer vorteilhaften Variante sind die röntgendichten Marker als Hohlzylinder oder Ringe aus Metall ausgestaltet, welche koaxial zur Katheterspitze angeordnet sind. Derartige Marker können direkt über die im Wesentlichen zylindrische Katheterspitze geschoben werden und beispielsweise durch Klemmen und/oder Stoffschluss befestigt werden. Es können aber prinzipiell auch anders geformte Marker verwendet werden, falls dies zweckdienlicher ist. Insbesondere abgerundete Marker sind vorteilhaft. Des Weiteren lassen sich beispielsweise auch mehrere Marker in Form von kleinen Keilen, welche zur Katheterspitze hin verjüngend ausgebildet sind, verwenden. Auch geeignet sind linsenförmige Plättchen, welche in die Katheterspitze eingebettet und beispielsweise angeklebt werden.

Bevorzugt sind die Hohlzylinder oder Ringe aus Metall in einem Bereich vor dem Verbindungsbereich des Katheters und der Katheterspitze angeordnet. Da der Verbindungsbereich zwischen Katheter und Katheterspitze meist etwas dicker ausgeformt ist als der vordere Teil der Katheterspitze, bildet der Verbindungsbereich einen hinteren Anschlag für die Hohlzylinder oder Ringe aus Metall. Mit Vorteil weisen die Hohlzylinder oder Ringe aus Metall daher einen Aussendurchmesser auf, welcher höchstens gleich dem Aussendurchmesser an der Rückseite der Katheterspitze ist. Damit ist sichergestellt, dass die Marker die Katheterspitze im Durchmesser nicht überragen, was wiederum die Einführbarkeit verbessert.

Mit Vorteil weisen die röntgendichten Marker in einer Längsrichtung der Katheterspitze eine Breite von insgesamt nicht mehr als 0,7 mm auf. Dickere Breiten sind auch möglich, reduzieren aber die Flexibilität der erfindungsgemässen Katheterspitzen.

Sämtliche hier beschriebenen Katheterspitzen können im vorderen Bereich zusätzlich gebogen sein. Damit wird insbesondere ein Einführen in abzweigende Hohlorgane erleichtert.

Aus der nachfolgenden Detailbeschreibung und der Gesamtheit der Patentansprüche ergeben sich weitere vorteilhafte Ausführungsformen und Merkmalskombinationen der Erfindung.

### Kurze Beschreibung der Zeichnungen

Die zur Erläuterung der Ausführungsbeispiele verwendeten Zeichnungen zeigen:
- Fig. 1: eine teilweise aufgeschnittene Seitenansicht einer Katheterspitze,
- Fig. 2: eine Ansicht von vorne auf die Katheterspitze gemäss Fig. 1,
- Fig. 3: eine Ansicht von vorne auf die Katheterspitze gemäss Fig. 1 mit einem zweiten freien Lumen,
- Fig. 4: eine Ansicht von vorne auf die Katheterspitze gemäss Fig. 1 ohne Führungsdrahtlumen,
- Fig. 5: eine abgeschrägte Katheterspitze gemäss Fig. 1, und
- Fig. 6: eine Variante von Fig. 5,
- Fig. 7: eine Katheterspitze mit Ausbauchung im vorderen Bereich im Querschnitt,
- Fig. 8: eine Katheterspitze mit schraubenförmiger Oberfläche im vorderen Bereich im Querschnitt,
- Fig. 9: eine Katheterspitze mit schraubenförmiger Oberfläche im Verbindungsbereich im Querschnitt,
- Fig. 10: eine Katheterspitze mit einem Marker im Querschnitt.

Grundsätzlich sind in den Figuren gleiche Teile mit gleichen Bezugszeichen versehen.

### Wege zur Ausführung der Erfindung

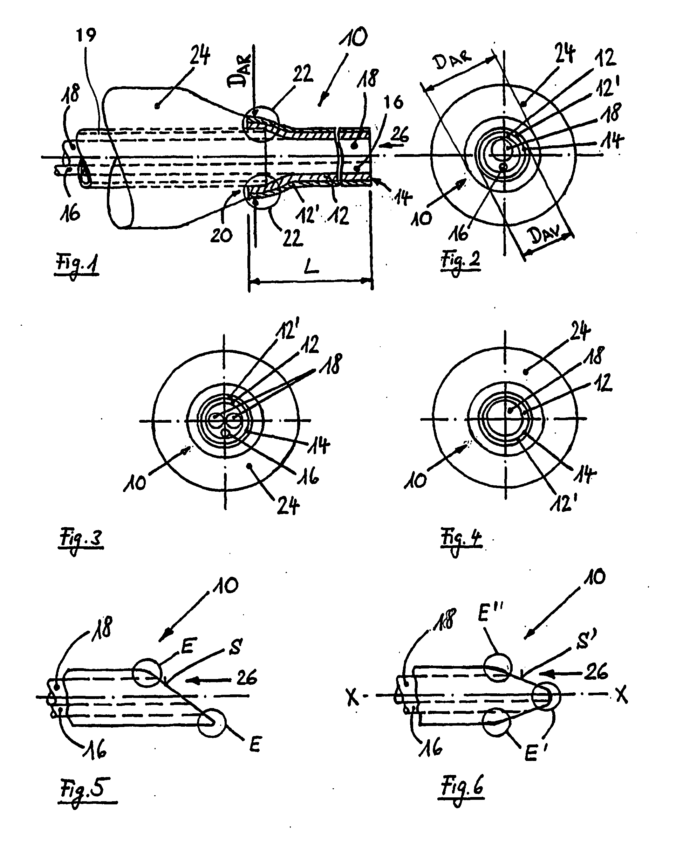

Fig. 1 zeigt eine bevorzugte Ausführungsvariante der Katheterspitze 10, welche dreischichtig ausgeführt ist und eine Länge L von etwa 4 mm aufweist. Es sind zwei zylindrisch ineinander liegende Schichten 12, 12' dargestellt, welche durch eine dünne Bindezwischenschicht 14 getrennt sind. Die Bindezwischenschicht 14 ist der Übersichtlichkeit wegen nur als einfacher Strich dargestellt und ist etwa 2 µm dick. Wie erwähnt besteht dabei die innere Schicht 12 aus einem Werkstoff hoher Druckfestigkeit, in der dargestellten Variante aus High Density Polyethylen (HDPE), die Bindezwischenschicht 14 aus einem Werkstoff mit guten Adhäsionseigenschaften, im Beispiel aus Plexar, und die äussere Schicht 12' aus einem flexiblen, aber nur gering dehnbaren Werkstoff, im Beispiel Polyamid (PA).

Weiter ist in Fig. 1 ein Führungsdrahtiumen 16 und ein freies Lumen 18 dargestellt. Auf die Darstellung des Führungsdrahtes ist der Übersichtlichkeit wegen verzichtet worden, er ist allgemein bekannt. Mit dem freien Lumen 18 ergibt wie erwähnt die Möglichkeit, ein flüssiges und/oder gasförmiges Medium vor der Katheterspitze 10 in das betreffende Organ einzuführen beziehungsweise zu applizieren.

Die Rückseite 20 der Katheterspitze 10 ist in einem Verbindungsbereich 22 auf einen Katheter 24 aufgesetzt, nach einer nicht dargestellten Variante eingesetzt. Die Katheterspitze 10, der Katheter 24 und der zylindrische Katheterschaft 19 sind hierbei miteinander unter Druck verschweisst (pressgeschmolzen). Der Aussendurchmesser DAR an der Rückseite 20 der Katheterspitze 10 beträgt hierbei nach dem Aufsetzen auf den nur ansatzweise dargestellten Katheter 24 0,51 mm.

Fig. 2 zeigt eine Ansicht auf die Vorderseite 26 der Katheterspitze 10 gemäss Fig. 1. Dabei wird die dreischichtige Ausführung der Katheterspitze 10 sowie das Führungsdrahtlumen 16 und das freie Lumen 18 verdeutlicht. Der Aussendurchmesser D_{AV} an der Vorderseite 26 der Katheterspitze 10 beträgt hierbei 0,45 mm.

Fig. 3 und Fig. 4 zeigen als Ausführungsvarianten ebenfalls eine Ansicht auf die Vorderseite 26 der Katheterspitze 10 gemäss Fig. 1. Im Unterschied zu Fig. 1 und 2 weist die Fig. 3 hierbei ein zweites freies Lumen 18 auf, wodurch zwei unterschiedliche Medien direkt vor der Katheterspitze 10 in das betreffende Organ eingeführt werden können. In Fig. 4 wird im Unterschied zu Fig. 1 und auf ein Führungsdrahtlumen 16 verzichtet. Wie erwähnt ist diese Variante besonders gut geeignet, wenn die Katheterspitze zum Beispiel in ein Organ eingeführt wird, welches vergleichsweise kurz und/oder geradlinig ist. Entsprechend grösser ist nun der Querschnitt des freien Lumens 18 gestaltet. Das freie Lumen 18 kann in dieser Ausführungsform beispielsweise zum Einführen einer Stammzelle genutzt werden.

Fig. 5 zeigt die Vorderseite. 26 der Katheterspitze 10 gemäss Fig. 1, wobei die Katheterspitze 10 in der dargestellten Variante eine nach vorne einseitig zulaufende Schräge S aufweist. Diese Ausführungsform ist dann besonders vorteilhaft, wenn der Katheter 24 mit der aufgesetzten Katheterspitze 10 in ein Organ mit kleinem Querschnitt eingeführt werden muss, beispielsweise ein Herzkranzgefäss. Die Schräge S weitet das Organ während des Einführvorgangs kontinuierlich auf. Um das betreffende Organ vor Verletzungen zu schützen, ist die Schräge S an deren Enden E abgerundet, insbesondere am spitzen Ende.

Fig. 6 stellt eine Schräge S' dar, welche beidseitig, symmetrisch zur Längsmittelachse X-X, nach vorne zuläuft. Auch die Schräge S' ist an deren Enden E' abgerundet.

Alle Ausführungsvarianten sind im Bereich der Rückseite 20 und im Bereich der Vorderseite 26 beziehungsweise der Schräge S, S' angeschmolzen. Ein Anschmelzen dieser Stellen bewirkt einen aussergewöhnlich gleichmässigen, glatten Übergang zwischen den unterschiedlichen Querschnitten und vermindert durch eine verbesserte Anschmiegbarkeit der Katheterspitze 10 den Einführwiderstand in ein Organ.

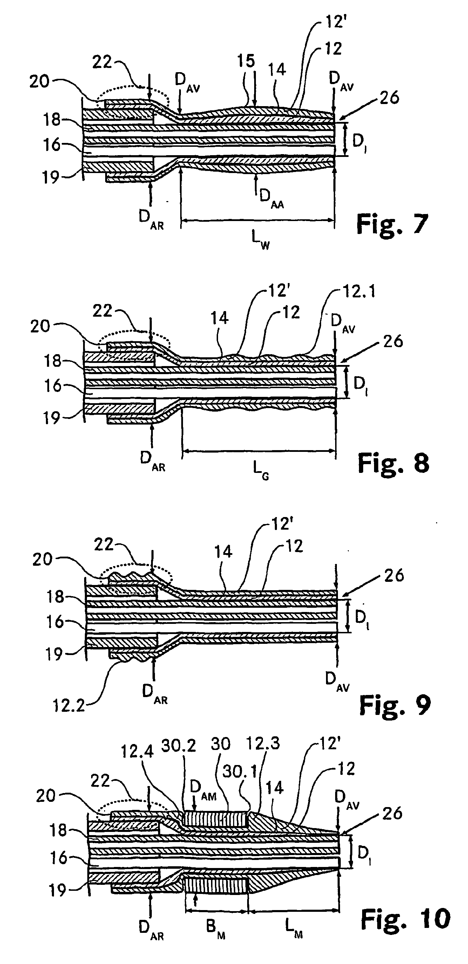

Fig. 7 zeigt einen Querschnitt durch eine weitere bevorzugte Ausführungsform einer erfindungsgemässen Katheterspitze 10. Im Unterschied zur Fig. 1 weisen die drei übereinander liegenden Schichten 12, 12', 14 im Bereich zwischen dem Verbindungsbereich 22 und der Vorderseite 26 eine symmetrisch nach aussen gewölbte Ausbuchtung 15 mit einer gewölbten Länge L_{W} in Längsrichtung der Katheterspitze von 3,5 mm auf. Dies wird dadurch erreicht, dass die übereinander liegenden Schichten 12, 12' im Bereich der gewölbten Ausbuchtung 15 dicker ausgestaltet sind, als in den anderen Bereichen der Katheterspitze 10. Der Aussendurchmesser D_{AV} der Katheterspitze 10 vor der gewölbten Ausbuchtung 15 und der vordere Aussendurchmesser D_{AV} an der Vorderseite 26 der Katheterspitze 10 sind dabei gleich gross und betragen 0.47 mm. Damit ist das Verhältnis von gewölbter Länge L_{W} zu Aussendurchmesser D_{AV} der Katheterspitze 10 gleich 7,45. Der Innendurchmesser D_{I} an der Vorderseite 26 beträgt 0,40 mm und ist über die gesamte gewölbte Länge L_{W} konstant. Im Bereich der Ausbuchtung 15 ist der Aussendurchmesser D_{AA} um ca. 15% grösser als der Aussendurchmesser D_{AV} an der Vorderseite 26 der Katheterspitze 10. Der Aussendurchmesser D_{AR} an der Rückseite 20 der Katheterspitze 10 im Verbindungsbereich 22 misst 0,55 mm.

Die in Fig. 8 dargestellte Ausführungsvariante einer Katheterspitze 10 weist im Unterschied zur Fig. 1 im Bereich zwischen dem Verbindungsbereich 22 und der Vorderseite 26 einen zylindrisch ausgeformten vorderen Teil auf, wobei die äussere Schicht 12 an der Oberfläche als Aussengewinde 12.1 ausgeformt ist. Die Länge L_{G} des Aussengewindes 12.1 beträgt 3,5 mm, bei einer Gewindesteigung von 1 mm. Der Innendurchmesser D_{I} im Bereich zwischen dem Verbindungsbereich 22 und der Vorderseite 26 beträgt konstant 0,40 mm, während der Aussendurchmesser in diesem Bereich durchschnittlich 0,47 mm misst.

Die Katheterspitze 10, welche in Fig. 9 im Querschnitt abgebildet ist, unterscheidet sich von der Variante in Fig. 1 dadurch, dass die äussere Schicht 12 im Verbindungsbereich 22 an der Rückseite 20 als Aussengewinde 12,2 ausgebildet ist. Der durchschnittliche Aussendurchmesser D_{AR} an der Rückseite 20 beträgt 0,55 mm. Der Innendurchmesser D_{I} im Bereich zwischen dem Verbindungsbereich 22 und der Vorderseite 26 beträgt 0,40 mm, während der Aussendurchmesser in diesem Bereich durchschnittlich 0,47 mm misst. Somit ist das Verhältnis von Aussendurchmesser D_{AV} zu Innendurchmesser D_{I} der Katheterspitze 10 gleich 1,175. Der durchschnittliche Aussendurchmesser D_{AR} an der Rückseite 20 ist verglichen mit dem Aussendurchmesser D_{AV} an der Vorderseite 26 um einen Faktor 1,17 grösser.

Fig. 10 zeigt einen Querschnitt durch eine weitere bevorzugte Ausführungsform einer erfindungsgemässen Katheterspitze 10. Im Unterschied zur Fig. 1 ist direkt vor dem Verbindungsbereich 22 aussen auf den drei Schichten 12, 12', 14 ein Platinring 30 als Marker koaxial auf der Katheterspitze 10 angeordnet. Der Platinring 30 weist einen Aussendurchmesser D_{AM} von 0,55 mm und einen Innendurchmesser von 0,47 mm auf. Seine Breite B_{M} in Längsrichtung der Katheterspitze 10 beträgt 0,62 mm. Der Aussendurchmesser D_{AR} an der Rückseite 20 des Katheters 10 im Verbindungsbereich 22 misst ebenfalls 0,55 mm. Die äussere Schicht 12' ist im Bereich 12,3 direkt vor dem Platinring 30 um 0,08 mm dicker ausgeformt, so dass die Vorderkante 30.1 des Platinrings vollständig von der äusseren Schicht 12' verdeckt ist. Die drei Schichten 12, 12', 14 sind zudem an der Aussenseite auf einer Länge L_{M} von 3 mm zur Vorderseite 26 hin konisch verjüngend ausgebildet. Damit ist die Vorderseite 30.1 des Platinrings 30 3 mm von der Vorderseite 26 bzw. der Spitze des Katheters entfernt. Der Aussendurchmesser D_{AV} der Katheterspitze 10 an der Vorderseite beträgt 0,41 mm und ist daher nahezu gleich dem Innendurchmesser D_{I} an dieser Stelle, welcher 0,40 mm misst. An der Hinterseite 30.2 des Platinrings sind sowohl die innere Schicht 12 als auch die äussere Schicht 12' dicker ausgestaltet, so dass die Hinterseite 30.2 des Platinrings 30 ebenfalls von der äusseren Schicht 12' verdeckt ist. Damit ist der Platinring 30 auf drei Seiten von der äusseren Schicht 12' umgeben und in diese eingebettet.

Bei dem in Fig. 7 gezeigten Ausführungsbeispiel ist es auch möglich, zusätzlich zur Ausbuchtung 15 die äussere Schicht 12 an der Oberfläche als Aussengewinde, wie bei Fig. 8 oder Fig. 9 beschrieben, auszubilden.

Des Weiteren können auch bei den in Fig. 1 bis 9 dargestellten Ausführungsbeispielen zusätzlich Marker angebracht werden, um eine Positionsbestimmung durch Röntgenabsorbtion zu ermöglichten.

Anstelle des in Fig. 10 dargestellten Platinring 30 können auch Marker aus einem anderen röntgendichten Material angebracht werden. Um die Flexibilität der Katheterspitze zu erhöhen, können auch mehrere nebeneinander liegende Platinringe von geringer Einzelbreite aber gleicher Gesamtbreite angebracht werden.

Prinzipiell ist es auch möglich, die Katheterspitzen mit einer Krümmung in Längsrichtung auszuformen. Dabei sind Krümmungen von 30-40° ideal und erlauben insbesondere ein einfacheres Abzweigen in verzweigten Hohlorganen.

Zusammenfassend ist festzustellen, dass die erfindungsgemässen Katheterspitzen im Vergleich zum Stand der Technik aufgrund der geringen Dimensionen und der optimierten Struktur wesentlich flexibler und gleichzeitig knickstabiler sind. Damit lassen sie sich kontrolliert und schonend durch problematische Verengungen auch in schwer zugängliche, enge Gefässe oder Hohlorgane einführen.

## Patentansprüche

1. Katheterspitze (10) mit einer Vorderseite (26) und einer Rückseite (20), welche auf einen Katheter (24) aufsetzbar ist, wobei die Katheterspitze (10) zwei im Wesentlichen zylindrisch ineinander liegende Schichten (12, 12') mit einer dünnen Bindezwischenschicht (14) umfasst, **dadurch gekennzeichnet, dass** die äussere Schicht (12) der Katheterspitze (10) im Bereich einer äusseren Oberfläche wenigstens in einem Teilbereich eine um die Katheterspitze (10) gewundene schraubenartige Struktur (12.1, 12.2) aufweist.

2. Katheterspitze (10) nach Anspruch 1, **dadurch gekennzeichnet, dass**
a) a) die innere Schicht (12) aus einem Werkstoff hoher Druckfestigkeit besteht, vorzugsweise aus High Density Polyethylen oder Teflon,
b) b) die Bindezwischenschicht (14) aus einem Werkstoff mit guten Adhäsionseigenschaften besteht, vorzugsweise aus Plexar, und/oder
c) c) die äussere Schicht (12') aus einem flexiblen, aber nur gering dehnbaren Werkstoff besteht, vorzugsweise aus Polyamid, Nylon und/oder Polyester.

3. Katheterspitze (10) nach Anspruch 1 oder 2, **dadurch gekennzeichnet, dass** die Bindezwischenschicht (14) eine Dicke von 1 bis 10 µm, vorzugsweise etwa 2 µm, aufweist.

4. Katheterspitze (10) nach einem der Ansprüche 1 bis 3, **dadurch gekennzeichnet, dass** sie eine Länge (L) von 0,5 bis 10 mm, insbesondere 3 bis 5 mm, aufweist.

5. Katheterspitze (10) nach einem der Ansprüche 1 bis 4, **dadurch gekennzeichnet, dass** sie nach vorne schräg zulaufend ausgebildet ist, wobei
a) ein Aussendurchmesser (D_{AV}) an der Vorderseite (26) 0,35 bis 0,5 mm, insbesondere etwa 0,45 mm, und/oder
b) ein Aussendurchmesser (D_{AR}) an der Rückseite (20) 0,45 bis 0,6 mm, insbesondere etwa 0,5 mm, beträgt.

6. Katheterspitze (10) nach einem der Ansprüche 1 bis 5, **dadurch gekennzeichnet, dass** die Katheterspitze (10) wenigstens ein freies Lumen (18) zum Einbringen eines flüssigen und/oder gasförmigen Mediums umfasst.

7. Katheterspitze (10) nach einem der Ansprüche 1 bis 6, **dadurch gekennzeichnet, dass** die Austrittsöffnung des freien Lumens (18) einen Durchmesser von 0,05 bis 0,42 mm aufweist, insbesondere etwa 0,35 mm.

8. Katheterspitze (10) nach einem der Ansprüche 1 bis 7, **dadurch gekennzeichnet, dass** sie mit einem über die Länge (L) abschnittsweise konstanten oder veränderlichen, kreisförmigen, ovalen und/oder elliptischen Querschnitt ausgebildet ist.

9. Katheterspitze (10) nach einem der Ansprüche 1 bis 8, **dadurch gekennzeichnet, dass** wenigstens eine Schicht (12, 12', 14) wenigstens teilweise gefärbt, reflektierend und/oder fluoreszierend ausgebildet ist.

10. Katheterspitze (10) nach einem der Ansprüche 1 bis 9, **dadurch gekennzeichnet, dass** sie weich und/oder im Bereich des Katheters (24) einschichtig ausgeführt ist.

11. Katheterspitze (10) nach einem der Ansprüche 1 bis 10, **dadurch gekennzeichnet, dass** in einem Verbindungsbereich (22) zwischen Katheterspitze (10) und Katheter (24), welcher vorzugsweise als Ballonkatheter und/oder als Monorail-Katheter ausgeführt ist,
- die Katheterspitze (10) stirnflächig zum Katheter (24) verbindbar ausgeformt ist, und/oder
- die Katheterspitze (10) in den Katheter (24) und/oder der Katheter (24) in die Katheterspitze (10) einschiebbar ist.

12. Katheterspitze (10) nach Anspruch 11, **dadurch gekennzeichnet, dass** der Verbindungsbereich (22) der Katheterspitze (10) verschweissbar, verklebbar, pressbar und/oder pressschmelzbar ausgeführt ist.

13. Katheterspitze (10) nach einem der Ansprüche 1 bis 12, **dadurch gekennzeichnet, dass** ein Bereich der Rückseite (20) und/oder ein Bereich der Vorderseite (26), vorzugsweise die Enden (E, E') einer Schräge (S, S'), angeschmolzen sind.

14. Katheterspitze (10), nach einem der Ansprüche 1 bis 13, **dadurch gekennzeichnet, dass** die Katheterspitze (10) In einem Bereich vor dem Verbindungsbereich (22) mit dem Katheter (24) eine gewölbte Ausbuchtung (15) aufweist, wobei die Ausbuchtung (15) bevorzugt tonnenartig ausgestaltet ist.

15. Katheterspitze (10) nach Anspruch 14, **dadurch gekennzeichnet, dass** ein Aussendurchmessar (D_{W}) Im Bereich der Ausbuchtung (16) 6-25 %**,** insbesondere 10-20 %, grössar ist als ein vorderer Aussendurchmesser (D_{AV}) der Katheterspitze (10) Im Bereich der Vorderseite (26) dar Katheterspitze (10).

16. Katheterspitze (10) nach einem der Ansprüche 1 - 15, **dadurch gekennzeichnet**, daas die schraubenlenförmige Struktur (12.1, 12.2) von der Vorderseite (26) beginnand in einer Richtung zur Rückseite (20) der Katherspitze (10) hin verläuft und bevorzugt eine Länge (Lₒ) von wenigstens 2 mm, insbesondere wenigstens 3 mm, aufweist.

17. Katheterspitze (10) nach einem der Ansprüche 1 - 16, **dadurch gekennzeichnet, dass** die schraubenertige Struktur (12.1, 12.2) eine Gewindesteigung von 0,1-5 mm, insbesondere 0,8-1,5 mm aufweist.

18. Katheterspitze (10) nach einem der Ansprüche 1-15, **dadurch gekennzeichnet, dass** die schraubenertige Struktur (12.1, 12,2) im Vorbindungsbereich (22) der Katheterspitze (10) und des Katheters (24) angebracht ist.

19. Katheterspitze (10), nach einem der Ansprüche 1 bis 18, **dadurch gekennzeichnet, dass** an der Katheterspitze (10) ein oder mehrere röntgendichte Marker (30) angebracht sind.

20. Katheterspitze (10) nach Anspruch 19, **dadurch gekennzeichnet, dass** die Marker (90) in die äussere Schicht (12) eingebeitet sind.

21. Katheterspitze (10) nach Anspruch 19 bis 20, **dadurch gekennzeichnet, dass** ein Abstand der Marker (30) von der Vorderseits (26) der Katheterspitze (10) wenigstens gleich 5 Mal, bevorzugt 10 Mal, so gross ist, wie ein vorderer Innendurchmesser (D_{I}) an der Vorderseite (26) der Katheterspitze (10).

22. Katheterspitze (110) nach einem der Ansprüche 19 bis 21, **dadurch gekennzeichnet dass** die röntgendichten Marker (30) Platin enthalten und bevorzugt ein Voluman von 0,10-0,20 mm³ aufweisen.

23. Katheterspitze (10) nach einem der Ansprüche 19. bis 22, **dadurch gekennzeichnet, dass** die röntgendichten Marker (30) eis Hohlzylinder oder Ringe aus Metall ausgesteltet sind, weiche koaxial zur Katheterspitze (10) angeordnet sind.

24. Katheterspitze (10) nach Anspruch 23, **dadurch gekennzeichnet dass** Hohlzylinder oder Ringe aus Metall in einem Bereich vor dem Verbindungsbereich (22) des Katheters (24) und der Katheterspitze (10) angeordnet sind und einem Aussendurchmesser (D_{AM}) aufweisen, welcher höchstens gleich dem Aussendurchmesser (D_{AR}) an der Rückseite (20) der Ketheterspitze (10) Ist.

25. Katheterspitze (10) nach einem der Ansprüche 19 bis 20, **dadurch gekennzeichnet, dass** die röntgendichten Marker (30) In einer Längsrichtung der Katheterspitze (10) eine Breite (Bᵤ) von insgesamt nicht mehr als 0,7 mm aufweisen.

## Claims

1. Catheter tip (10) with a front side (26) and a rear side (20), which can be placed on a catheter (24), the catheter tip (10) comprising two layers (12, 12') lying substantially cylindrically one inside the other with a thin intermediate connecting layer (14), **characterised in that** the outer layer (12) of the catheter tip (10) has, in the region of an outer surface, at least in a part region, a helicoidal structure (12.1, 12.2) wound around the catheter tip (10).

2. Catheter tip (10) according to claim 1, **characterised in that**
a) the inner layer (12) consists of a material with high compressive strength, preferably high density polyethylene or Teflon,
b) the intermediate connecting layer (14) consists of a material with good adhesion properties, preferably Plexar, and/or
c) the outer layer (12') consists of a flexible, but only slightly extendable material, preferably polyamide, nylon and/or polyester.

3. Catheter tip (10) according to claim 1 or 2, **characterised in that** the intermediate connecting layer (14) has a thickness of 1 to 10 µm, preferably about 2 µm.

4. Catheter tip (10) according to any one of claims 1 to 3, **characterised in that** it has a length (L) of 0.5 to 10 mm, in particular 3 to 5 mm.

5. Catheter tip (10) according to any one of claims 1 to 4, **characterised in that** it tapers obliquely to the front, wherein
a) an external diameter (D_{AV}) on the front side (26) is 0.35 to 0.5 mm, in particular about 0.45 mm, and/or
b) an external diameter (D_{AR}) on the rear side (20) is 0.45 to 0.6 mm, in particular about 0.5 mm.

6. Catheter tip (10) according to any one of claims 1 to 5, **characterised in that** the catheter tip (10) comprises at least one free lumen (18) for the introduction of a liquid and/or gaseous medium.

7. Catheter tip (10) according to any one of claims 1 to 6, **characterised in that** the outlet opening of the free lumen (18) has a diameter of 0.05 to 0.42 mm, in particular about 0.35 mm.

8. Catheter tip (10) according to any one of claims 1 to 7, **characterised in that** it is formed with a circular, oval and/or elliptical cross section that is variable or constant in sections over the length (L).

9. Catheter tip (10) according to any one of claims 1 to 8, **characterised in that** at least one layer (12, 12', 14) is at least partially coloured, reflective and/or fluorescent.

10. Catheter tip (10) according to any one of claims 1 to 9, **characterised in that** it is single-layered in the region of the catheter (24) and/or soft.

11. Catheter tip (10) according to any one of claims 1 to 10, **characterised in that** in a connection region (22) between the catheter tip (10) and catheter (24), which is preferably formed as a balloon catheter and/or as a monorail catheter,
- the catheter tip (10) is formed so that it can be connected at the end face to the catheter (24) and/or
- the catheter tip (10) can be inserted into the catheter (24) and/or the catheter (24) can be inserted in the catheter tip (10).

12. Catheter tip (10) according to claim 11, **characterised in that** the connection region (22) of the catheter tip (10) can be welded, glued, pressed and/or press-melted.

13. Catheter tip (10) according to any one of claims 1 to 12, **characterised in that** one region of the rear side (20) and/or one region of the front side (26), preferably the ends (E, E') of a bevel (S, S'), are melted on.

14. Catheter tip (10) according to any one of claims 1 to 13, **characterised in that** the catheter tip (10) has an arched bulge (15) in a region in front of the connection region (22) to the catheter (24), the bulge (15) preferably being barrel-like.

15. Catheter tip (10) according to claim 14, **characterised in that** an external diameter (D_{AA}) in the region of the bulge (15) is 5 to 25 %, in particular 10 to 20 % greater than a front external diameter (D_{AV}) of the catheter tip (10) in the region the front side (26) of the catheter tip (10).

16. Catheter tip (10) according to any one of claims 1 to 15, **characterised in that** the helical line-shaped structure (12.1, 12.2), extends beginning from the front side (26) in one direction to the rear side (20) of the catheter tip (10) and preferably has a length (L_{G}) of at least 2 mm, in particular at least 3 mm.

17. Catheter tip (10) according to any one of claims 1 to 16, **characterised in that** the helicoidal structure (12.1, 12.2) has a thread pitch of 0.1 to 5 mm, in particular 0.8 to 1.5 mm.

18. Catheter tip (10) according to any one of claims 1 to 15, **characterised in that** the helicoidal structure (12.1, 12.2) is attached in the connection region (22) of the catheter tip (10) and the catheter (24).

19. Catheter tip (10) according to any one of claims 1 to 18, **characterised in that** one or more X-ray-tight markers (30) are attached to the catheter tip (10).

20. Catheter tip (10) according to claim 19, **characterised in that** the markers (30) are embedded in the outer layer (12).

21. Catheter tip (10) according to claim 19 to 20, **characterised in that** a spacing of the markers (30) from the front side (26) of the catheter tip (10) is at least equal to 5 times, preferably 10 times as large as a front internal diameter (D_{I}) on the front side (26) of the catheter tip (10).

22. Catheter tip (10) according to any one of claims 19 to 21, **characterised in that** the X-ray-tight markers (30) contain platinum and preferably have a volume of 0.10 to 0.20 mm³.

23. Catheter tip (10) according to any one of claims 19 to 22, **characterised in that** the X-ray-tight markers (30) are configured as hollow cylinders or rings made of metal which are arranged coaxially with respect to the catheter tip (10).

24. Catheter tip (10) according to claim 23, **characterised in that** hollow cylinders or rings made of metal are arranged in a region in front of the connection region (22) of the catheter (24) and the catheter tip (10) and have an external diameter (D_{AM}), which is at most equal to the external diameter (D_{AR}) on the rear side (20) of the catheter tip (10).

25. Catheter tip (10) according to any one of claims 19 to 20, **characterised in that** the X-ray-tight markers (30), in a longitudinal direction of the catheter tip (10), have a width (B_{M}) of a total of no more than 0.7 mm.

## Revendications

1. Embout (10) de cathéter enfilable sur un cathéter (24) et présentant un côté avant (26) et un côté arrière (20), l'embout (10) de cathéter comprenant deux couches (12, 12') placées à l'intérieur l'une de l'autre de façon sensiblement cylindrique et une mince couche intermédiaire d'adhésif (14), **caractérisé en ce que** la couche externe (12) de l'embout (10) de cathéter présente au moins dans une zone partielle, dans la zone de sa surface externe, une structure filetée (12.1, 12.2) enroulée autour de l'embout (10) de cathéter.

2. Embout (10) de cathéter selon la revendication 1, **caractérisé en ce que**
a) la couche interne (12) se compose d'une matière à haute résistance à la compression, de préférence un polyéthylène à haute densité ou du téflon,
b) la couche intermédiaire d'adhésif (14) se compose d'une matière à bonnes propriétés d'adhérence de préférence du Plexar, et/ou
c) la couche externe (12') se compose d'une matière flexible mais peu extensible, de préférence un polyamide, un nylon, et/ou un polyester.

3. Embout (10) de cathéter selon la revendication 1 ou 2, **caractérisé en ce que** l'épaisseur de la couche intermédiaire d'adhésif (14) est de 1 à 10 µm, de préférence environ 2 µm.

4. Embout (10) de cathéter selon la revendication 1, 2 ou 3, **caractérisé en ce que** sa longueur (L) est de 0,5 à 10 mm, en particulier 3 à 5 mm.

5. Embout (10) de cathéter selon l'une quelconque des revendications précédentes, **caractérisé en ce que** sa structure est en sifflet vers l'avant d'une manière telle que
a) son diamètre externe (D_{AV}) sur le côté avant (26) est de 0,35 à 0,5 mm, en particulier environ 0,45 mm, et
b) son diamètre externe (D_{AR}) sur le côté arrière (20) est de 0,45 à 0,6 mm, en particulier environ 0,5 mm.

6. Embout (10) de cathéter selon l'une quelconque des revendications précédentes, **caractérisé en ce que** l'embout (10) de cathéter comprend au moins un lumen libre (18) pour l'introduction d'un milieu liquide et/ou gazeux.

7. Embout (10) de cathéter selon l'une quelconque des revendications précédentes, **caractérisé en ce que** le diamètre de l'orifice de sortie du lumen libre (18) est de 0,05 à 0,42 mm, en particulier environ 0,35 mm.

8. Embout (10) de cathéter selon l'une quelconque des revendications précédentes, **caractérisé en ce que** sa section transversale est circulaire, ovale et/ou elliptique, et variable ou constante par segments sur la longueur (L).

9. Embout (10) de cathéter selon l'une quelconque des revendications précédentes, **caractérisé en ce qu'**au moins une couche (12, 12', 14) est réalisée au moins en partie de façon colorée, réfléchissante et/ou fluorescente.

10. Embout (10) de cathéter selon l'une quelconque des revendications précédentes, **caractérisé en ce qu'**il est de structure monocouche dans la zone du cathéter (24) et/ou souple.

11. Embout (10) de cathéter selon l'une quelconque des revendications précédentes, **caractérisé en ce que**, dans une zone de connexion (22) entre l'embout (10) de cathéter et le cathéter (24) qui est de préférence réalisée sous la forme d'un cathéter à ballon ou d'un cathéter monorail,
- la surface frontale de l'embout (10) de cathéter est configurée de façon connectable avec le cathéter (24), et/ou
- l'embout (10) de cathéter est insérable dans le cathéter (24) et/ou le cathéter (24) est insérable dans l'embout (10) de cathéter

12. Embout (10) de cathéter selon la revendication 11, **caractérisé en ce que** la zone de connexion (22) de l'embout (10) de cathéter peut être soudée, collée, emboutie et/ ou soudée par pression.

13. Embout (10) de cathéter selon l'une quelconque des revendications précédentes, **caractérisé en ce qu'**une zone du côté arrière (20) et/ou une zone du côté avant (26), de préférence les extrémités (E, E') d'un sifflet (S, S'), sont fondues.

14. Embout (10) de cathéter selon l'une quelconque des revendications précédentes, **caractérisé en ce que** l'embout (10) de cathéter présente, dans une zone située en avant de la zone de connexion (22) avec le cathéter (24), un renflement bombé (15), ce renflement bombé (15) étant de préférence en forme de barillet.

15. Embout (10) de cathéter selon la revendication 14, **caractérisé en ce que** le diamètre externe (data) dans la zone du renflement (15) est supérieur de 5 à 25%, en particulier de 10 à 20% au diamètre externe (D_{AV}) avant de l'embout (10) de cathéter dans la zone du côté avant (26) de l'embout (10) de cathéter.

16. Embout (10) de cathéter selon l'une quelconque des revendications précédentes, **caractérisé en ce que** la structure filetée (12.1, 12.2) s'étend depuis le côté avant (26) en direction du côté arrière (20) de l'embout (10) de cathéter et que sa longueur (L_{G}) est de préférence d'au moins 2 mm, en particulier au moins 3 mm.

17. Embout (10) de cathéter selon l'une quelconque des revendications précédentes, **caractérisé en ce que** le pas de filetage de la structure filetée (12.1, 12.2) est de 0,1 à 5 mm, en particulier 0,8 à 1,5 mm.

18. Embout (10) de cathéter selon l'une quelconque des revendications 1 à 15, **caractérisé en ce que** la structure filetée (12.1, 12.2) est formée dans la zone de connexion (22) de l'embout (10) de cathéter et du cathéter (24).

19. Embout (10) de cathéter selon l'une quelconque des revendications précédentes, **caractérisé en ce qu'**un ou plusieurs marqueurs opaques en radiographie (30) sont disposés sur l'embout (10) de cathéter.

20. Embout (10) de cathéter selon la revendication 19, **caractérisé en ce que** les marqueurs (30) sont enrobés dans la couche externe (12).

21. Embout (10) de cathéter selon la revendication 19 ou 20, **caractérisé en ce que** la distance entre les marqueurs (30) et le côté avant (26) de l'embout (10) de cathéter est au moins égale à 5 fois, de préférence 10 fois, le diamètre interne avant (D_{I}) sur le côté avant (26) du cathéter (10).

22. Embout (10) de cathéter selon la revendication 19, 20 ou 21, **caractérisé en ce que** les marqueurs (30) opaques en radiographie contiennent du platine et que leur volume est de préférence de 0,10 à à 0,20 mm³,

23. Embout (10) de cathéter selon l'une quelconque des revendications 19 à 22, **caractérisé en ce que** les marqueurs (30) opaques en radiographie consistent en cylindres creux ou en bagues métalliques qui sont coaxiaux avec l'embout (10) de cathéter.

24. Embout (10) de cathéter selon la revendication 23, **caractérisé en ce que** des cylindres creux ou des bagues métalliques sont disposés dans une zone en avant de la zone de connexion (22) du cathéter (24) et de l'embout (10) de cathéter et que leurs diamètres externes (D_{AM}) sont au plus égaux au diamètre externe (D_{AR}) sur le côté arrière (20) de l'embout (10) de cathéter.

25. Embout (10) de cathéter selon la revendication 19 ou 20, **caractérisé en ce que** la largeur (B_{M}) des marqueurs (30) opaques en radiographie, dans la direction longitudinale de l'embout (10) de cathéter, ne dépasse pas au total 0,7 mm.
